# EUROPEAN PATENT APPLICATION

(11) **EP 3 761 011 A1**
(43) Date of publication of application: **06.01.2021**
(21) Application number: 19757866.9
(22) Date of filing: 26.02.2019
(51) Int. Cl.: G01N 21/84, B07C 5/342, F21V 7/09, G01N 21/85, F21Y 115/10

(54) **ILLUMINATING DEVICE FOR SORTING MACHINE OR INSPECTING MACHINE**

(30) Priority: 26.02.2018 JP 2018031560
(71) Applicant: Satake Corporation, Chiyoda-ku Tokyo 101-0021 (JP)
(72) Inventor: KAWAMURA Yoichi, Tokyo 101-0021 (JP)
(74) Representative: Regimbeau
(86) International application number: PCT/JP2019/007217
(87) International publication number: WO 2019/164009

(57) **Abstract**

It is intended to provide an illuminating device for supplying luminous energy to a to-be-detected object, using one illumination light source to create a detection image which is bright in its entirety and free from any shadow area. The light sources necessary to illuminate the object from thereabove and therebelow are commonalized, wherein light from the common illumination light source is divided by the reflectors, such that it becomes upward illumination and downward illumination, to illuminate the object with the illuminations. Optical paths of a part of light rays from the illumination light source are reflected by a refractive mirror, such that the part of the light rays is incident on at least one of the reflectors. This makes it possible to reduce the number of the illumination light sources without causing a decrease in the luminous energy, thereby realizing a reduction in size of the entire illuminating device.

## Description

### TECHNICAL FIELD

The present invention relates to a color sorting machine or the like for sorting or inspecting granular objects such as grains or resin pellets, based on colors, and particularly to an illuminating device for, when an optical detector images granular objects as to-be-detected objects, illuminating the to-be-detected objects.

### BACKGROUND ART

Heretofore, there has been known a sorting machine for separating granular-type objects to be detected, e.g., grains such as rice grains or soybeans, resin pellets, or coffee grains, into a first group falling within a given criterion regarding color, shape or the like, and a second group falling outside the given criterion, or for sorting and removing a foreign object mixed in the objects to be detected (see the following Patent Document 1).

As described in the Patent Document 1, an optical detection unit of a conventional sorting machine is operable to image objects to be detected (e.g., grains) which are moving obliquely downwardly in a falling manner on a tilt table (hereinafter referred to as "chute") disposed in the sorting machine. This optical direction unit comprises at least a visible light camera, and a visible light source such as a fluorescent lamp (in some cases, further comprises a near-infrared camera, and a near-infrared light source such as a halogen lamp). In order to clearly detect the entirety of each grain without occurrence of shadow in an image of the grain detected by the optical detection unit, illumination from upper and lower sides on each of the front and rear sides is needed as well as imaging the grain from both front and rear sides thereof on the basis of a falling trajectory (wherein the term "front and rear sides" is expressed in the sense that, when viewing a rice grain from one camera, a surface of the rice grain facing the camera is a front object surface, and the opposite surface is a rear object surface; this will also be applied to the following). That is, illumination from total four positions is desired. For this reason, an illumination light source of the optical detection unit was needed to be provided in a number of four (two on the front side, and two on the rear side).

Further, when the value of luminous energy (quantity of light) of one of the grains received by the camera falls outside a given proper range, the grain is estimated to be a defective grain, and blown off by blast from an injector nozzle, thereby being separated from non-defective grains. In order for imaging of each grain, the falling trajectory of the grain is predicted, and an illuminating device is disposed such that the object surface of the grain is illuminated with light. However, if the object surface deviates from a predicted position, or if a part of the grain is not illuminated with light under the influence of an incident angle of the illumination light, an unclear detection image will be undesirably created. This directly leads to a factor causing deterioration in detection accuracy as one index of performance of a sorting machine for removing a defective grain while determining whether each grain is non-defective or defective. Studying where the light sources of the illuminating device should be installed in the optical detection unit to allow the entire object surface of each grain to be illuminated with light emitted from the light sources, reliably and efficiently, is an important matter in design and production of the sorting machine.

### CITATION LIST

### [Parent Document]

Patent Document 1: JP 2007-283204A

### SUMMARY OF INVENTION

### [Technical Problem]

Assume a case where it is attempted to illuminate each grain by arranging the illumination light sources, respectively, at four positions, as in the optical detection unit of the conventional sorting machine. In this case, for example, when each of the illumination light sources is composed of an LED board in which a plural number (N) of LED chips are arranged in conformity to the width of the chute, 4N LED chips are needed. Further, in order to electronically detect an LED burn-out in each of the LED boards, a plurality of driving circuits proportional to the number of the LED chips are needed, leading to an increase in cost. If the number of the illumination light sources, and the number of the LED chips in each of the illumination light sources, can be reduced, the cost of the LED chips can be suppressed. Furthermore, it becomes possible to incorporate, in the sorting machine, a low-cost LED burn-out detection mechanism in which the driving circuits may be simplified according to a less number of the LED chips.

After thousands per second of grains are vigorously fed onto the chute from a to-be-detected object transfer chute, a part of the grains fall down while bouncing on the chute. As a matter of course, the object surface of each of such a part of grains is not on the same plane as that of the object surface of each of the remaining grains falling down without bouncing. On the other hand, the conventional optical detection unit is optically designed such that, assuming that the object surfaces of the grains are uniformly on the same plane, light from the illumination light sources is focused on each of the assumed object surfaces. Thus, there arose a situation where any grain falling down while bouncing on the chute is not sufficiently illuminated with the illumination light. As a result, there was a problem that a detection image becomes dark due to insufficiency in luminous energy, or a shadow which would never occur if sufficient luminous energy is supplied to the entire grain appears in the image, leading to deterioration in detection accuracy.

In view of the above technical problems, the present invention is directed to provide a sorting machine or an inspecting machine comprising an illuminating device which is configured to supply sufficient luminous energy for each object to be detected while reducing the number of illumination light sources, to create a detection image which is bright in its entirety, and has less shadowed area.

### [Solution to Technical Problem]

In the present application, an illuminating device used for a sorting machine or an inspecting machine for distinguishing, based on an image of a to-be-detected object detected by an optical detection unit, whether the to-be-detected object is non-defective or defective, comprising: a light source unit having a light source configured to emit illumination light; and a plurality of reflectors configured to reflect light rays from the light source unit toward the to-be-detected object, wherein the plurality of reflectors include a first reflector and a second reflector which are arranged across an optical axis of the optical detection unit, wherein at least one of the first reflector and the second reflector is a concave curved mirror, and , wherein light rays from the same light source unit are reflected by the first reflector and the second reflector, such that the light rays take respective optical paths toward the to-be-detected object to illuminate the to-be-detected object from thereabove and therebelow.

In the present application, an illuminating device used for a sorting machine or an inspecting machine for distinguishing, based on an image of a to-be-detected object detected by optical detection unit, whether the to-be-detected object is non-defective or defective, comprising:a light source unit having a light source configured to emit illumination light; and a plurality of reflectors configured to reflect light rays from the light source unit toward the to-be-detected object, wherein the plurality of reflectors include a first reflector and a second reflector which are arranged across an optical axis of the optical detection unit, wherein each of the first reflector and the second reflector is a planar mirror, and further comprises a refractive mirror disposed on optical paths of at least a part of the light rays emitted from the light source unit, wherein the refractive mirror has a first concave curved reflection surface and a second concave curved reflection surface, wherein: the first concave curved reflection surface is configured to bend the optical paths of at least the part of the light rays emitted from the light source unit to reflect at least the part of the light rays toward the first reflector; and the second concave curved reflection surface is configured to reflect, toward the second reflector, at least a part of light rays which do not reach the first concave curved reflection surface, among the light rays from the light source unit, whereby the light rays reflected by the first reflector and the second reflector take respective optical paths toward the to-be-detected object to illuminate the to-be-detected object from thereabove and therebelow.

### [Effect of Invention]

In the illuminating device of the present invention, a plurality of light sources are commonalized, which is necessary to illuminate the to-be-detected object from thereabove and therebelow on each of front and rear sides of the object on the basis of a falling trajectory of the object. A part of a group of light rays emitted from a single illumination light source, and a part of the remaining light rays, are reflected, respectively, by the first reflector and the second reflector. Light from the illumination light source can be reflected by the reflectors, such that it is divided into upward and downward illuminations, and then the divided light rays may illuminate the object. This makes it possible to reduce the number of the illumination light sources without causing extreme decrease in luminous energy as compared with conventional illuminating devices, thereby contributing to a reduction in cost of the illuminating device as well as a sorting machine incorporating the illuminating device.

In the illuminating device of the present invention, optical paths of at least a part of the light rays from the illumination light source may be changed by the refractive mirror, such that at least the part of the light rays are received by at least one of the first reflector and the second reflector, so as to illuminate the to-be-detected object. This makes it possible to reduce an envelope size of the illuminating device to realize a reduction in size of the entire sorting machine.

In the illuminating device of the present invention, at least one of the first reflector and the second reflector may be a concave curved mirror (a concave cylindrical mirror having a curvature only in one direction). In this case, the concave curved surface of the concave curved mirror may be composed of a plurality of concave curved surface portions having different curvatures, or may be a concave free-curved surface (generally called a free-curved surface, an anamorphic aspheric surface having no rotational symmetry, or a high-order rotational aspheric surface). The local curvature radius continuously and arbitrarily changes depending on position and orientation on the concave curved surface. Alternatively, at least one of the first reflector and the second reflector may be a special concave curved mirror such as a mirror having a concave curved surface approximated by a plurality of planar surfaces. Light emitted from the illumination light source are directed toward the to-be-detected object according to a reflection angle based on a curvature at each position on the concave curved surface, so that the beams of light are focused such that a region of a width including the to-be-detected object becomes an illumination range. Thus, the object is present within the illumination range even if the falling trajectory of the object deviates from an assumed falling trajectory, so that it is possible to avoid a situation where a shadow arises in an image detected by a camera, and/or the image itself becomes dark. Therefore, the present invention may achieve high accuracy of detection.

### BRIEF DESCRIPTION OF DRAWINGS

FIG. 1 is a sectional schematic diagram showing a first embodiment of an illuminating device of the present invention.
FIG. 2 is a sectional schematic diagram showing a second embodiment of the illuminating device of the present invention.
FIG. 3A is a sectional schematic diagram showing a configuration in which a refractive mirror and a first reflector in the second embodiment are composed of a concave curved mirror and a planar mirror, respectively.
FIG. 3B is a sectional schematic diagram showing a configuration in which each of the refractive mirror and the first reflector in the second embodiment is composed of a concave curved mirror.
FIG. 4 is a sectional schematic diagram showing a third embodiment of the illuminating device of the present invention.
FIG. 5A is a sectional schematic diagram showing a configuration in which a refractive mirror in the third embodiment is composed of a plurality of concave curved mirrors.
FIG. 5B is a sectional schematic diagram showing a configuration in which the refractive mirror in the third embodiment is composed of a plurality of concave curved mirrors, and each of first and second reflectors in the third embodiment is composed of a planar mirror.
FIG. 6 is a sectional schematic diagram showing a fourth embodiment of the illuminating device of the present invention, wherein the refractive mirror and the first reflector in the second embodiment are formed as an integral structure.
FIG. 7 is a sectional schematic diagram showing a fifth embodiment of the illuminating device of the present invention, wherein the refractive mirror and the first reflector in the third embodiment are formed as an integral structure.
FIG. 8 is a sectional schematic diagram of a sorting machine, for explaining a relationship between a falling trajectory of a to-be-detected object and an optical path of light from an illumination light source.
FIG. 9 is a sectional schematic diagram showing a sixth embodiment of the illuminating device of the present invention.
FIG. 10 is a sectional schematic diagram showing a seventh embodiment of the illuminating device of the present invention.
FIG. 11 is a sectional schematic diagram showing an eighth embodiment of the illuminating device of the present invention.
FIG. 12 is a sectional schematic diagram showing a ninth embodiment in which a first reflector and a second reflector in the sixth embodiment are used in an illuminating device according to the first embodiment.
FIG. 13 is a diagram showing an optical path in a situation where reflected light by a first reflector and a second reflector is focused on a rear side of a to-be-detected object.
FIG. 14 is a diagram for explaining that, when using the first reflector and the second reflector in the sixth embodiment, the distribution of luminous energy can be controlled with respect to each incident angle of light rays toward a to-be-detected object, depending on a difference in surface curvature among concave curved surface portions.
FIG. 15 is a diagram showing one example in which a light-emitting face of an LED of an illumination light source is reflected on a detection image of a to-be-detected object having a lustrous or glossy surface.
FIG. 16 is a diagram showing a third embodiment of the illuminating device of the present invention, and one example in which a plurality of lines are provided on a concave curved surface of each of a first reflector and a second reflector.

### DESCRIPTION OF EMBODIMENTS

With reference to the drawings, embodiments of an illuminating device of the present invention in a case where the illuminating device is applied to a sorting machine will now be described.

As with the aforementioned Document 1, the sorting machine is configured such that the objects to be detected are supplied to a chute from thereabove to fall through the chute, and imaged by a camera (e.g., a line sensor camera) comprised in an optical detection unit including an illuminating device. The illuminating device are arranged on each or one of front and rear sides of the falling objects across the falling trajectories of the objects. After irradiating the objects with light emitted from an illumination light source of the illuminating device, reflected or transmitted light is imaged by the camera, thereby acquiring optical information of the objects. In the following embodiments, the objects to be sorted by the sorting machine are assumed to be rice grains. Since defective rice grains and foreign substances such as stones are included in harvested rice grains at a certain level, it is necessary to remove them. Thus, the sorting machine is configured to separate, based on the optical information, the objects into either rice grains satisfying a criterion or other objects (i.e., foreign substances).

The object determined to fall outside the criterion or to be a foreign substance will be conveyed to a pathway different from that for non-defective objects, by a force of air discharged from an injector nozzle.

The present invention is a technique relating, particularly, to the illuminating device among components of the sorting machine. The illuminating device which is configured to emit illumination light toward the objects for the non-defective or defective determination by the sorting machine comprises at least an illumination light source and a plurality of reflectors. It should be noted that, in the following description, details of the configuration and function of any component other than the illuminating device, i.e., any component which is not associated directly with the present invention, will be omitted for simplicity.

### < FIRST EMBODIMENT >

An illumination light source to be applied to an illuminating device according to the first embodiment is constructed by arranging a plurality of LED chips on a board in one line or in a planar pattern in conformity to the width of the chute. A plurality of LEDs of different colors may be used in a mixed manner. In another embodiment, any light emitter other than the LED chip may be used.

Each of a plurality of reflectors to be used in this embodiment is a concave curved mirror. Specifically, an ellipsoidal concave curved mirror having an ellipsoidal curvature is used. Here, it does not necessarily mean that a concave curved surface of the mirror is formed to have an ellipsoidal curvature, but may be formed to have a parabolic or hyperbolic curvature, e.g., when it is necessary to lower light collected to each object to be detected. Alternatively, the concave curved surface may be a free-curved surface capable of freely setting an incident angle, a collecting position and a state of light directed toward each object to be detected.

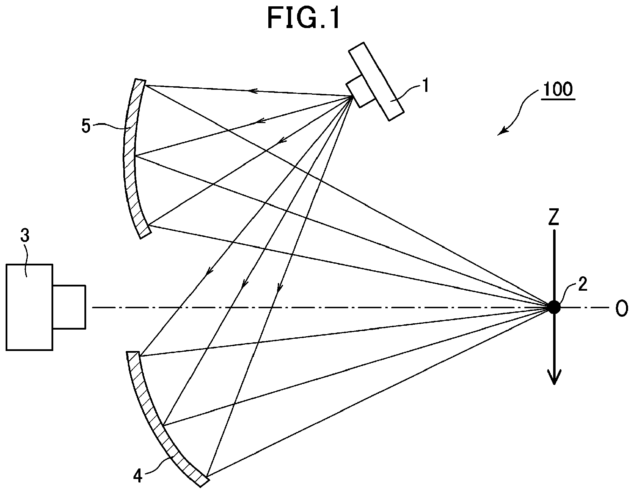

FIG. 1 is a schematic diagram of an illuminating device 100 according to the first embodiment, wherein solid lines are used to express what kind of optical paths are taken by light rays emitted from an illumination light source 1, so as to illuminate a rice grain 2 which is one of the to-be-detected objects.

As shown in FIG. 1, two reflectors 4, 5 are arranged between a camera 3 and the rice grain 2 and across an optical axis O connecting the camera 3 and the rice grain 2. As mentioned above, in this embodiment, each of the reflectors 4, 5 is a concave curved mirror. Here, for convenience sake, the concave curved mirror disposed below the optical axis O, and the concave curved mirror disposed above the optical axis O, will hereinafter be referred to, respectively, as "first reflector 4" and "second reflector 5". These correspond, respectively, to "first reflector" and "second reflector" recited in the appended claims.

The illumination light source 1 is disposed only on the side of the second reflector 5. That is, this illumination device is configured such that a group of light rays from the illumination light source 1 is incident on the first reflector 4 and the second reflector 5, instead of a configuration in which an illumination light source is present with respect to each of the reflectors, individually and independently,

Thus, a part of the light rays from the illumination light source 1 are reflected by the first reflector 4 to travel toward the rice grain 2, and a part of the remaining light rays are reflected by the second reflector 5 to travel toward the rice grain 2. Specifically, in the first embodiment illustrated in FIG. 1, the light rays reflected by the second reflector 5 will illuminate an upper half of the front side of the rice grain 2, and the light rays reflected by the first reflector 4 will illuminate a lower half of the front side of the rice grain 2. FIG. 1 shows only illumination of the front side of the rice grain 2. When illuminating the rear side of the rice grain 2, a similar illuminating device may be disposed on the rear side of the rice grain 2 across a falling trajectory Z of the rice grain 2.

In the first embodiment, the illuminating device 100 comprising a single illumination light source 1 simultaneously illuminates the upper half and lower half of one of the front and rear sides of the rice grain 2, so that luminous energy radiated from the illumination light source can be effectively utilized, thereby simply reducing the number of conventional illumination light sources to one-half. This makes it possible to reduce a cost required for the illuminating device.

FIG. 1 shows the configuration in which the illumination light source 1 is disposed on the side of the second reflector 5 located above the optical axis O. However, it should be understood that, even in an arrangement in which the illumination light source 1 and the reflectors 4, 5 are reversed symmetrically with respect to the optical axis O, a similar technical effect can be obtained.

### < SECOND EMBODIMENT >

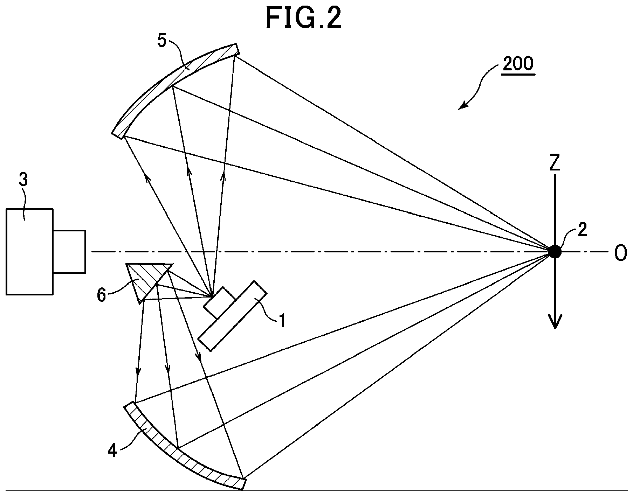

An illuminating device 200 according to the second embodiment further comprises a refractive mirror 6 as illustrated in FIG. 2. The functions of the remaining components are the same as those in the first embodiment, and therefore description thereof will be omitted.

In the second embodiment, the refractive mirror 6 is a planar mirror, and used to bend optical paths of light rays emitted from the illumination light source 1, such that the light rays reach the first reflector 4. For this purpose, differently from the arrangement in the first embodiment, the illumination light source 1 is disposed between the first reflector 4 and the second reflector 5, and the first reflector 4 (e.g., an ellipsoidal concave curved mirror) and the second reflector 5 are arranged such that they face each other, as shown in FIG. 2. As illustrated, instead of allowing a group of light rays from the illumination light source 1 to be incident on the first reflector 4, the part of the light rays first may travel toward the refractive mirror 6. As a result, the light rays reflected by the refractive mirror 6 become incident on the first reflector 4. The refractive mirror 6 is disposed in opposed relation to the illumination light source 1 and in opposed relation to the first reflector 4, so that the light rays from the illumination light source 1 can travel toward the rice grain 2 though a plurality of times of bending of the optical paths.

Here, it does not mean that optical paths of all the group of light rays from the illumination light source 1 are bent by the refractive mirror 6. As shown in FIG. 2, a part of the light rays from the illumination light source 1 are directly incident on the second reflector 5 (e.g., an ellipsoidal concave curved mirror), and only light rays to be made incident on the first reflector are bent by the refractive mirror 6. In FIG. 2, the refractive mirror 6 is disposed at a position below the optical axis O, and closer to the first reflector 4. However, depending a relationship with an emitting direction of light rays from the illumination light source 1, or a tilt of the refractive mirror 6, a part of or the entirety of the refractive mirror 6 may be disposed on the side of the second reflector 5 above the optical axis O.

In either case, in the illuminating device according to the second embodiment, the positions and orientations of the illumination light source 1, the refractive mirror 6 and the first and second reflectors 4, 5 are adjusted such that a part of the light rays from the illumination light source 1 are incident on the refractive mirror 6, and subsequently bent by the refractive mirror 6 to travel toward the first reflector 4, while at least a part of the remaining light rays from the illumination light source 1 are directly incident on the second reflector 5.

FIG. 2 shows the configuration in which the illumination light source 1 and the refractive mirror 6 are arranged at positions below the optical axis O and closer to the first reflector 4. However, it should be understood that, even in an arrangement in which the illumination light source 1, the reflectors 4, 5 and the refractive mirror 6 are reversed symmetrically with respect to the optical axis O, a similar technical effect can be obtained.

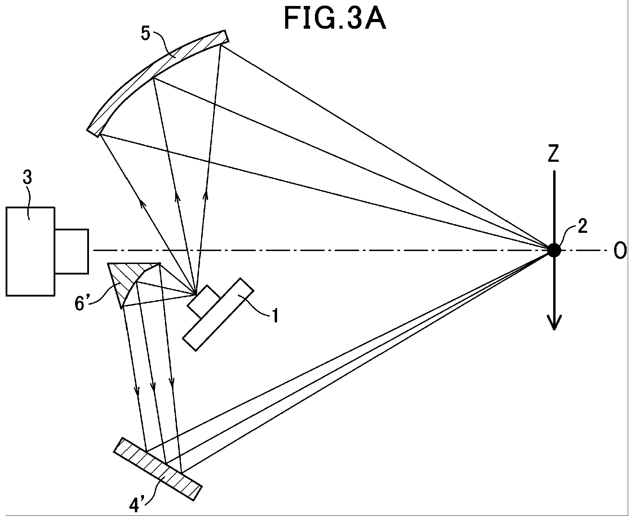

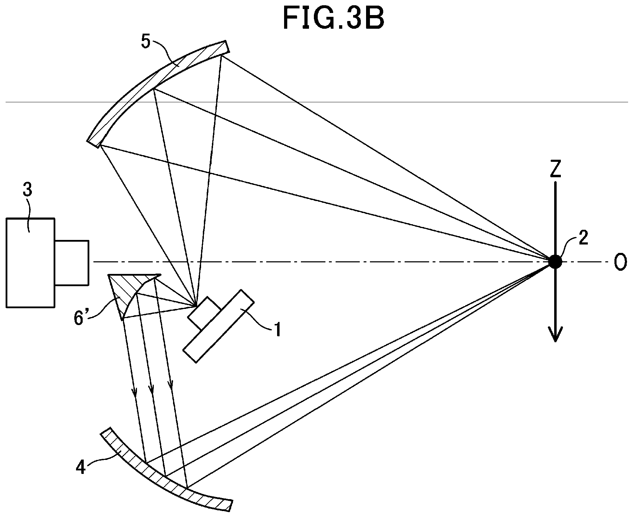

Further, in the second embodiment, the planar mirror is employed as the refractive mirror 6. However, it does not necessarily mean that the planar mirror has to be the planar mirror. For example, a concave curved-shaped refractive mirror 6' may be employed which is capable of allowing a group of light rays from the illumination light source 1 to travel separately in respective directions toward the first reflector 4 and the second reflector 5, in the same manner as that in the configuration using the planer mirror. In this case, the first reflector may be a reflector 4' composed of a planar mirror, as shown in FIG. 3(A), or may be a reflector 4 composed of a concave curved mirror, as shown in FIG. 3(B).

In a case where, in each of the group of upper optical paths and the group of lower optical paths each extending from the illumination light source 1 to the rice grain 2, the light rays from the illumination light source 1 are reflected only once by the concave curved mirror (or the concave curved-shaped refractive mirror), the concave curved mirror (or the concave curved-shaped refractive mirror) is preferably an ellipsoidal concave curved mirror. On the other hand, in a case where the light rays are reflected twice, a parabolic concave curved mirror is preferably used as the concave curved mirror or the concave curved-shaped refractive mirror. In this case, parallel light rays are formed through reflection by the first parabolic concave curved mirror, and then focused toward the rice grain through reflection by the second parabolic concave curved mirror.

Thus, in the configuration of FIG. 3(A) using the first reflector 4' composed of a planar mirror, the concave curved-shaped refractive mirror 6' is preferably composed by employing an ellipsoidal concave curved mirror. Further, the second reflector 5 on which light rays reflected from the refractive mirror 6' are not incident is also preferably composed by employing an ellipsoidal concave curved mirror. The configuration of FIG. 3(B) uses not only the concave curved-shaped refractive mirror 6' but also the first reflector 4 composed of a concave curved mirror. Thus, each of the refractive mirror 6' and the first reflector 4 is preferably composed by employing a parabolic concave curved mirror.

### < THIRD EMBODIMENT >

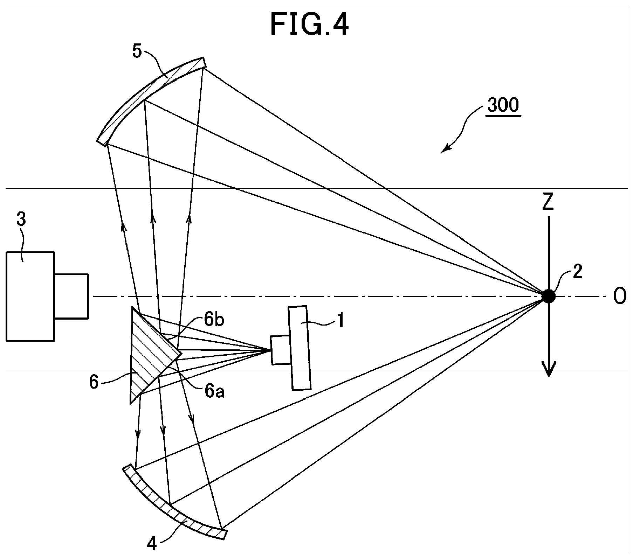

As shown in FIG. 4, an illuminating device 300 according to the third embodiment is characterized in that the refractive mirror 6 has a plurality of reflection surfaces 6a, 6b. As illustrated, a part of the light rays emitted from the illumination light source 1, whose optical paths are bent by the first reflection surface 6a, are reflected by the first reflector 4 which is a concave curved mirror (e.g., an ellipsoidal concave curved mirror), and then the reflected light rays become illumination light which illuminates the rice grain 2 from therebelow. Similarly, a part of the remaining light rays whose optical paths are bent by the second reflection surface 6b are reflected by the second reflector 5 which is a concave curved mirror (e.g., an ellipsoidal concave curved mirror), and then the reflected light rays become illumination light which illuminates the rice grain 2 from thereabove.

That is, the refractive mirror 6 in the second embodiment illustrated in FIG. 2 is configured to bend a part of the light rays from the illumination light source 1 by the single reflection surface, such that the part of the light rays travel toward the first reflector 4, whereas the refractive mirror 6 in the third embodiment is configured to bend the light rays from the illumination light source 1 by not only the first reflection surface 6a having a function similar to that of the single reflection surface, but also the second reflection surface 2b, such that the light rays travel toward the first reflector 4 and the second reflector 5.

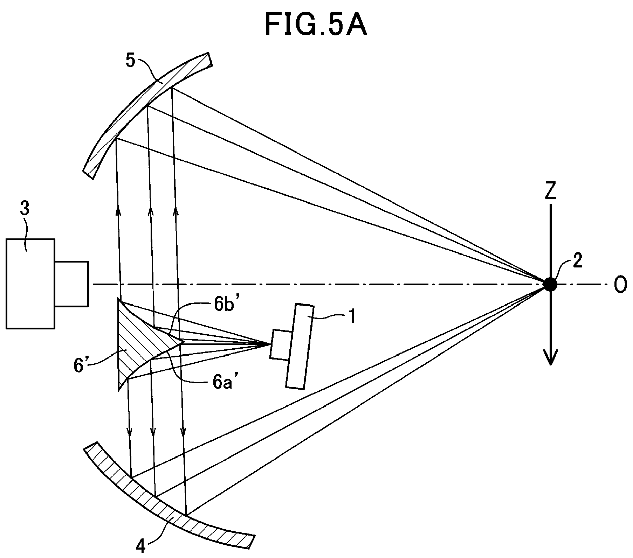

In FIG. 4, each of the first reflection surface 6a and the second reflection surface 6b of the refractive mirror 6 is shown as a planar reflecting mirror. However, as described in the second embodiment, there is no limitation that the refraction surface has to be a planar reflection surface, and the refraction surface can be a concave curved-shaped reflection surface. FIG. 5(A) shows a reflective mirror 6' (reflection surfaces 6a', 6b') as an example in which each of the two reflection surfaces of the refractive mirror 6 in the illuminating device 300 illustrated in FIG. 4 is modified such that it is composed of a concave curved mirror.

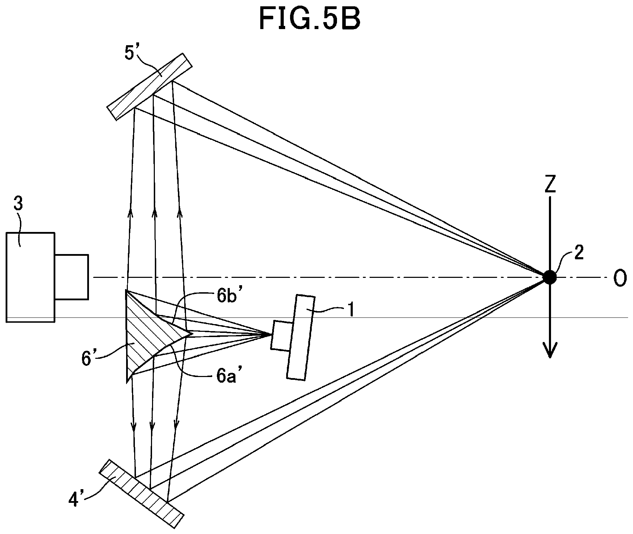

A further modification is shown in FIG. 5(B). In this modification, although each of the two reflection surfaces 6a', 6b' of the refractive mirror 6' is composed of a concave curved mirror as with the modification in FIG. 5(A), each of the first reflector 4 and the second reflector 5 each composed of a concave curved mirror is replaced, respectively, with a first reflector 4' and a second reflector 5' each composed of a planar mirror. This configuration also makes it possible to illuminate the rice grain 2 with light rays reflected by the two reflectors from thereabove and therebelow.

Here, for the same reason as that in FIGS. 3(A) and 3(B), each of the two reflection surfaces 6a', 6b' of the refractive mirror 6' and the first and second reflectors 4, 5 illustrated in DIG. 5(A) is preferably composed by employing a parabolic concave curved mirror. In FIG. 5(B), wherein each of the first reflector 4 and the second reflector 5 is a planar mirror, each of the two reflection surfaces 6a', 6b' of the refractive mirror 6' is preferably composed by employing an ellipsoidal concave curved mirror.

Further, a part of or the entirety of the refractive mirror 6 or 6' may be disposed on the side of the second reflector 5 or 5' above the optical axis O, as with the second embodiment. Further, even in an arrangement in which the illumination light source 1, the refractive mirror 6 or 6', the first reflector 4 or 4' and the second reflector 5 or 5' are reversed symmetrically with respect to the optical axis O, a similar technical effect can be obtained, as with the aforementioned embodiments.

### < FOURTH EMBODIMENT >

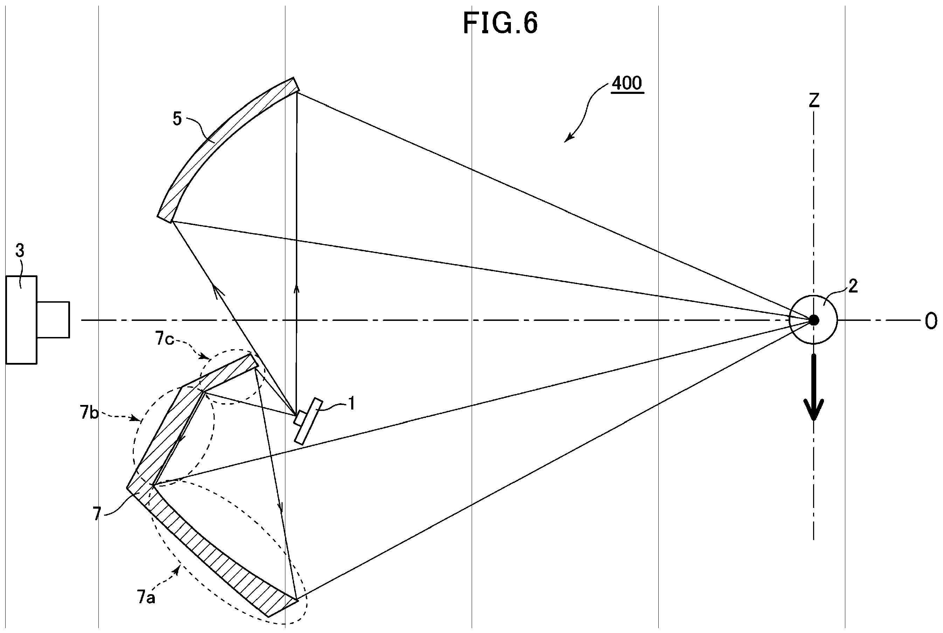

As shown in FIG. 6, an illuminating device 400 according to the fourth embodiment is characterized in that the refractive mirror 6 and the first reflector 4 in the second embodiment illustrated in FIG. 2 are formed as an integral structure. Giving an explanation in comparison with the second embodiment, a reflection surface portion 7c and a reflection surface portion 7a of a first reflector 7 illustrated in FIG. 6 are equivalent, respectively, to the refractive mirror 6 and the first reflector 4 in the second embodiment. The remaining components are the same as those in each of the first to third embodiments, and therefore description thereof will be omitted.

A part of the light rays emitted from the illumination light source 1 toward the reflection surface portion 7c of the first reflector 7 are reflected such that optical paths thereof are changed toward the reflection surface portion 7a. Light rays further reflected by the reflection surface portion 7a travel toward the rice grain 2 and become illumination light which illuminates the rice grain 2 from therebelow. At least a part of the remaining light rays which do not travel toward the reflection surface portion 7c are directly incident on the second reflector 5, and the resulting reflected light rays become illumination light which illuminates the rice grain 2 from thereabove, as with the second embodiment.

As one modification of the illuminating device in FIG. 6, the first reflector 7 may be disposed such that a part of or the entirety thereof may be located on the side of the second reflector 5 above the optical axis O, as with the aforementioned embodiments. Further, even in an arrangement in which the illumination light source 1, the first reflector 7 and the second reflector 5 are reversed symmetrically with respect to the optical axis O, a similar technical effect can be obtained, as with the aforementioned embodiments.

### < FIFTH EMBODIMENT >

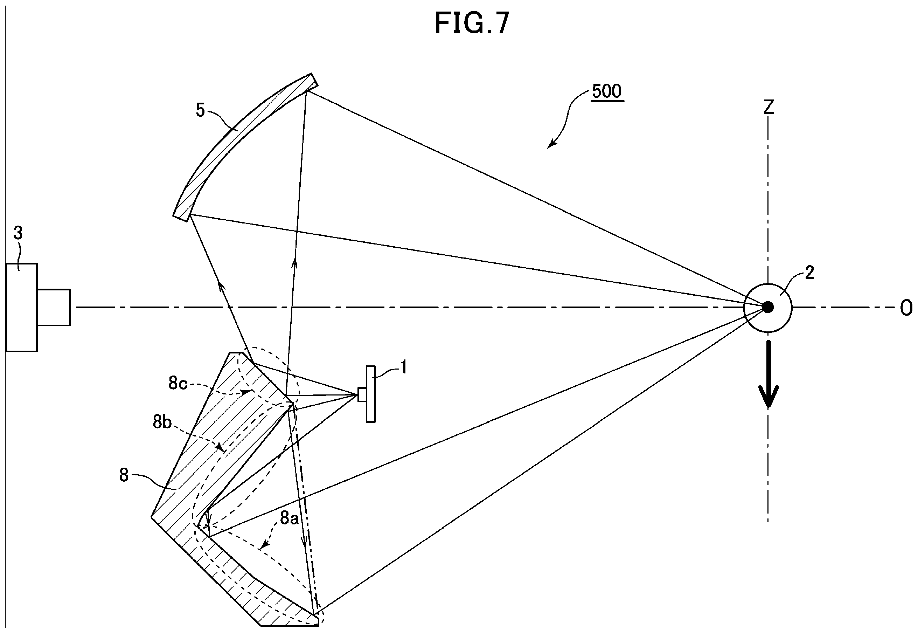

As shown in FIG. 7, an illuminating device 500 according to the fifth embodiment is characterized in that the refractive mirror 6 and the first reflector 4 in the third embodiment illustrated in FIG. 4 are formed as an integral structure. Giving an explanation in comparison with the third embodiment, two reflection surface portions 8b, 8c of a first reflector 8 illustrated in FIG. 7 are equivalent to the refractive mirror 6 having the reflection surfaces 6a, 6b in the third embodiment, and a reflection surface portion 8a of the first reflector 8 is equivalent to the first reflector 4 in the third embodiment. The remaining components are the same as those in each of the aforementioned embodiments, and therefore description thereof will be omitted. It should be understood that examples of a modification of the fifth embodiment include a configuration in which the refractive mirror 6' and the first reflector 4 or 4' in FIGS. 5(A) and 5(B) showing the modifications of the third embodiment are formed as an integral structure, although it is not illustrated.

A part of the light rays emitted from the illumination light source 1 toward the reflection surface portion 8b of the first reflector 8 are reflected such that optical paths thereof are changed toward the reflection surface portion 8a. Light rays further reflected by the reflection surface portion 8a travel toward the rice grain 2 and become illumination light which illuminates the rice grain 2 from therebelow. A part of the remaining light rays are reflated by the reflection surface portion 8c, such that optical paths thereof are changed toward the second reflector 5, and light rays reflected by the second reflector 5 become illumination light which illuminates the rice grain 2 from thereabove, as with the third embodiment.

Even in an arrangement in which the illumination light source 1, the first reflector 8 and the second reflector 5 are reversed symmetrically with respect to the optical axis O, a similar technical effect can be obtained, as with the aforementioned embodiments. Further, as one modification of the illuminating device in FIG. 7, the first reflector 8 may be disposed such that a part of or the entirety thereof may be located on the side of the second reflector 5 above the optical axis O, as with the aforementioned embodiments.

The fourth embodiment and the fifth embodiment are merely described as embodiments focusing on the point that a refractive mirror for distributing a group of light rays from the illumination light source 1 to a plurality of reflectors arranged above and below or across the optical axis O of the camera, and one of the reflectors, are formed as an integral structure. Thus, the reflection surface portion 7c or the set of reflection surface portions 8b, 8c having a function equivalent to that of the refractive mirror 6 or 6' may be in the form of not only a planar mirror but also a concave curved mirror, as with the second and third embodiments. Further, it should be noted that specific examples of the reflection surface portion 7a or 8a having a function equivalent to that of the first reflector 4 or 4', and the second reflector 5, include both a planar mirror and a concave curved mirror. Furthermore, the first reflector 7 or 8 may be disposed such that a part of or the entirety thereof may be located on the side of the second reflector 5 above the optical axis O, and, an arrangement in which the illumination light source 1, the first reflector 7 or 8 and the second reflector 5 are reversed symmetrically with respect to the optical axis O may be employed, as with the aforementioned embodiments.

In the illuminating devices described in the second to fifth embodiments, the single illumination light source 1 functions as a common light source for simultaneously illuminating the upper half and the lower half of one of the front and rear sides of the rice grain 2, as with the illuminating device 100 according to the first embodiment. Thus, luminous energy radiated from the single illumination light source can be effectively utilized, thereby simply reducing the number of conventional illumination light sources to one-half. This makes it possible to reduce a cost required for the illuminating device.

Further, the illumination light source 1 is disposed between (inward of) the two reflectors, instead of being disposed outward of the reflectors. Thus, the illuminating device can be configured to effectively realize a further reduction in size of the entire sorting machine, as compared to the first embodiment.

### < SIXTH EMBODIMENT >

Next, a case will be described in which a concave curved mirror is used as the aforementioned first or second reflector, and a concave curved surface thereof is configured to spread a focusing position in a front-rear direction without focusing light on one spot. Such a mirror surface shape can be applied to not only the first and second concave curved mirrors 4, 5 but also the reflection surface portions 7a, 8a of the reflectors 7, 8 in the aforementioned embodiments.

The following description will be made based on an example in which the technical idea of the sixth embodiment is applied to the configuration of the illuminating device 200 according to the second embodiment.

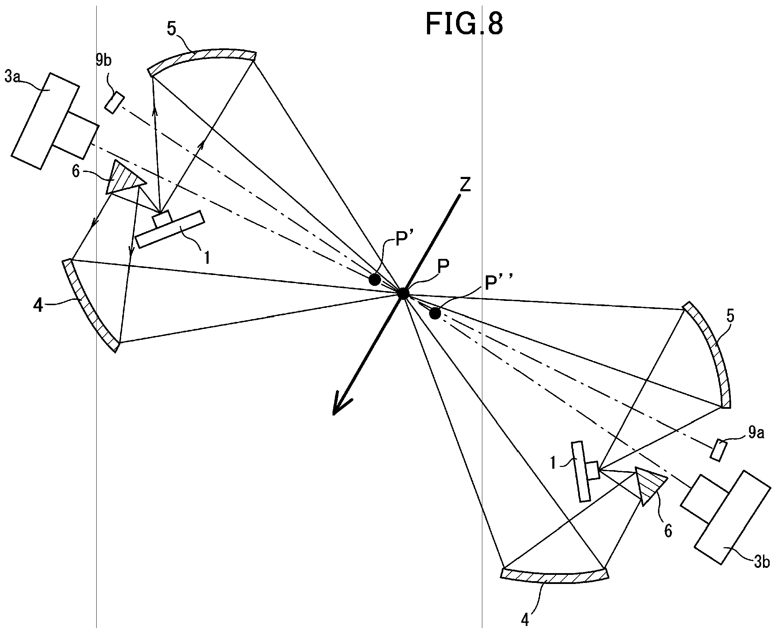

As shown in FIG. 8, in the sorting machine, a rice grain 2 moving along a falling trajectory Z while falling through the chute is imaged by two cameras 3 (front camera 3a and a rear camera 3b) from front and rear sides with respect to the rice grain 2. Assume that among a large number of rice grains, the rice grain 2 bounces on the chute. In this situation, during imaging of the front side of the rice grain by the front camera 3a, the rice grain 2 can be imaged at a detection position P' offset on the side of the front camera 3a or a detection position P" offset on the side of the rear camera 3b, with respect to an ideal detection position P at which an optical axis O of the front camera 3a intersects the falling trajectory Z (see FIG. 8). Here, the falling trajectory Z of the center of the rice grain is illustrated on the assumption that the object surface of the rice grain is the center of the rice grain, because the rice grain is very small. In FIG. 8, each of the reference signs 9a, 9a denotes a background for one of the cameras opposed thereto.

Generally, on the assumption that the ideal detection position P is coincident with the object surface of the rice grain 2, i.e., the center of the rice grain, a group of light rays from the illumination light source is focused to illuminate the object surface of the rice grain 2. Thus, when the level of deviation toward the front camera 3a is large during imaging of the front side of the rice grain by the camera 3a, the object surface can have an area which is not present on optical paths of light rays reflected by the first reflector 4 to illuminate the rice grain from therebelow, and an area which is not present on optical paths of light rays reflected by the second reflector 5 to illuminate the rice grain from thereabove. For example, the rice grain 2 deviating to the position P' illustrated in FIG. 8 is not illuminated with light.

There is also a possibility that the rice grain undesirably deviates to the position P" on the side of the rear camera 3b. In this situation, when the level of deviation is large, the object surface of the rice grain also fails to be present on optical paths of light rays reflected by the reflectors, and the rice grain is not illuminated with light. The problem during imaging of the front side of the rice grain by the front camera 3a is also applied to imaging of the rear side of the rice grain by the rear camera 3b.

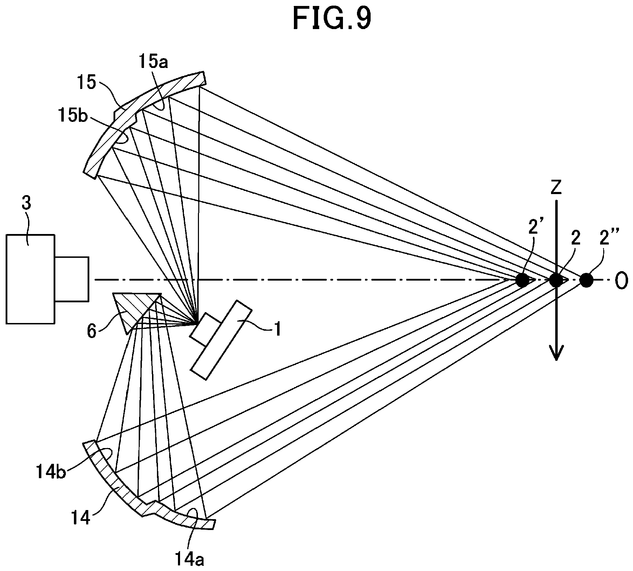

The sixth embodiment illustrated in FIG. 9 has been conceived to solve the influence on a detection image arising from a positional deviation of the object surface caused by a phenomenon that a to-be-detected object falls down while bouncing on the chute, or the like. Each of a first reflector 14 and a second reflector 15 in the illuminating device according to the sixth embodiment has a plurality of concave curved surface portions (e.g., two ellipsoidal concave curved surfaces) which are different in terms of curvature. Specifically, the plurality of concave curved surface portions comprises at least a first concave curved surface portion defined by a first curvature, and a second concave curved surface portion defined by a second curvature. The remaining components are the same as those in the first embodiment. Thus, the following description will be made about an effect of the concave curved mirror having the "plurality of concave curved surface portions".

The sixth embodiment is characterized in that the reflectors 14, 15 each having the plurality of concave curved surface portions are used to create a width in a focusing region, as shown in FIG. 9.

As is indicated by the cross-section of the first reflector 14 illustrated in FIG. 9, the first reflector 14 has a first concave curved surface portion 14a and a second concave curved surface portion 14b. Similarly, the second reflector 15 has a first concave curved surface portion 15a and a second concave curved surface portion 15b. Each of the first concave curved surface portion 14a and the second concave curved surface portion 14b of the first reflector 14 is configured to reflect light rays from the illumination light source 14a to illuminate the rice grain from therebelow. However, a focusing position of light rays reflected by the first concave curved surface portion 14a is not identical to a focusing position of light rays reflected by the second concave curved surface portion 14b. This is just because the surface curvature defining the first concave curved surface portion 14a is different from the surface curvature defining the second concave curved surface portion 14b.

For example, instead of focusing, on one spot, all of the light rays reflected by the first concave curved surface portion 14a to illuminate the rice grain from therebelow, the surface curvature of the first concave curved surface portion 14a is designed such that the reflected light rays are focused over a given range on the side rearward of the ideal detection position P of the rice grain 2 (in FIG. 9, in a direction coming away from the camera 3). Similarly, the surface curvature of the second concave curved surface portion 14b is designed such that the light rays reflected by the second concave curved surface portion 14b are also focused over a given range on the side forward of the ideal detection position P of the rice grain 2 (in FIG. 9, in a direction coming close to the camera 3). With regard to illumination for illuminating the rice grain from thereabove, the surface curvatures of the second reflector 15 are designed such that each set of light rays reflected by a respective one of the first concave curved surface portion 15a and the second concave curved surface portion 15b of the second reflector 15 are focused over a given range on the side rearward or forward of the ideal detection position P of the rice grain 2.

FIG. 9 shows an example in which the illumination range by the first concave curved surface portion 14a does not overlap the illumination range by the second concave curved surface portion 14b. However, the two illumination ranges may partially overlap each other. That is, the curvatures of the two concave curved surface portions may be adjusted such that the sets of light rays reflected by the first concave curved surface portion and the second concave curved surface portion partially overlay each other to illuminate a part of object surfaces of the rice grain 2 and a rice grain 2' or 2" with the reflected light from the two concave curved surface portions. Further, there is a possibility that light rays reflected by the first concave curved surface portion 14a are focused over a given range on the side forward of the ideal detection position P of the rice grain 2, and light rays reflected by the second concave curved surface portion 14b are focused over a given range on the side rearward of the ideal detection position P of the rice grain 2. This depends on the surface curvatures of the two concave curved surface portions.

The same is applied to the first concave curved surface portion 15a and the second concave curved surface portion 15b.

By controlling the focusing position of each of the first reflector 14 and the second reflector 15 having a focusing property, it becomes possible to realize illumination covering the entire front half of a rice grain, not only when the rice grain is moving just along an ideal falling trajectory, but also when the rice grain is falling down while deviating from the falling trajectory Z in the front-rear direction, like the rice grain 2' or 2". Although FIG. 9 shows only a region on the side of the front camera 3a, the same is completely applied to illumination for the entire rear half of the rice grain by the rear camera 3b. Thus, description thereof will be omitted.

Meanwhile, when the rice grain 2 bounces on the chute, how much the rice grain 2 bounces to become the rice grain 2' or 2" (determination of a divergence α between the position P of the rice grain 2 and the position P' or P" of the rice grain 2' or 2") is not accurately applicable to all rice grains falling down while bouncing. However, it can be theoretically calculated from the weight, falling velocity and others of a rice grain, or an expected value thereof can be derived based on an actual observation. Then, based on the divergence α, the surface curvature of the concave curved surface portions may be determined.

### < SEVENTH EMBODIMENT >

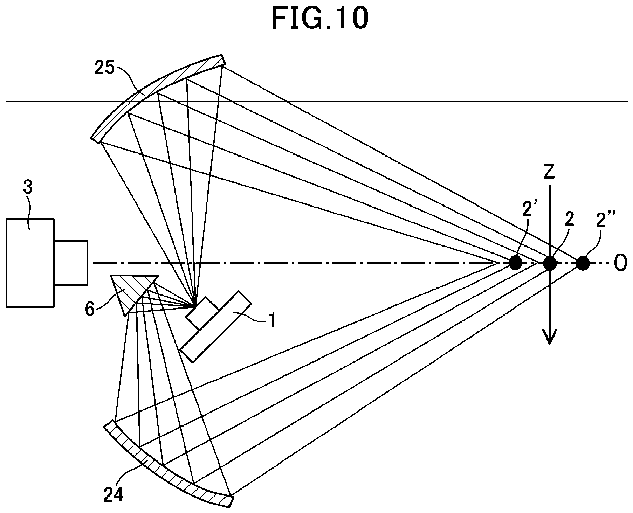

The concept of controlling the focusing position using a first reflector 24 and a second reflector 25 in the seventh embodiment illustrated in FIG. 10 is basically the same as that in the sixth embodiment. A difference is that the shape of each of the first and second reflectors 14, 15 in the sixed embodiment is a concave curved shape formed by combining a plurality of concave curved surface portions having different curvatures, and each of the concave curved surface portions is designed such that the focusing position thereof is located forward or rearward of the rice grain, whereas each of the first and second reflectors 24, 25 in the seventh embodiment is formed to have a concave free-curved shape, and the width of the focusing region is created by the curvature at an arbitrary position on a concave free-curved surface thereof.

### < EIGHTH EMBODIMENT >

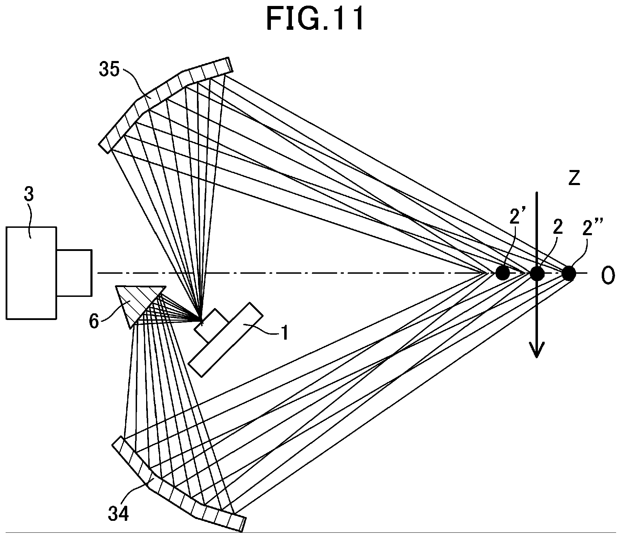

The concept of controlling the focusing position using a first reflector 34 and a second reflector 35 in the eighth embodiment illustrated in FIG. 11 is basically the same as that in the sixth embodiment. A difference is that the shape of each of the first and second reflectors 14, 15 in the sixed embodiment is a concave curved shape formed by combining a plurality of concave curved surface portions having different curvatures, and each of the concave curved surface portions is designed such that the focusing position thereof is located forward or rearward of the rice grain, whereas, in each of the first and second reflectors 34, 35 in the eighth embodiment, the width of the focusing region is created by forming the concave curved surface into a mirror surface shape approximated by a plurality of planar surfaces.

### < NINTH EMBODIMENT >

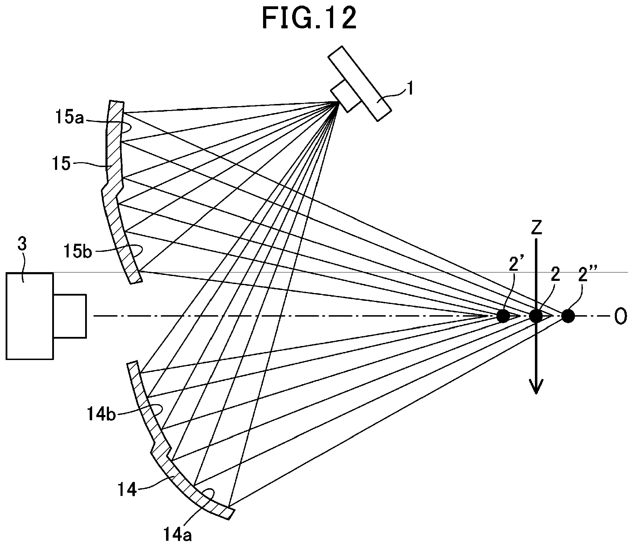

FIG. 12 shows optical paths of light rays from an illumination light source in one example where the technical idea regarding the first and second reflectors 14, 15 (reflectors each having a plurality of concave curved surface portions which are different in terms of curvature) in the sixth embodiment is applied to the illuminating device according to the first embodiment. It should be understood that this technical idea is also applicable to: the second reflector 5 in FIG. 3(A) and the first and second reflectors 4, 5 in FIG. 3(B) which are the modifications of the second embodiment; and the first and second reflectors 4, 5 in each of the configurations illustrated in FIG. 4 as the third embodiment and FIG. 5(A) as the modification thereof.

As described in the sixth to ninth embodiments, when the first reflector 14, 24 or 34 and the second reflector 15, 25 or 35, it becomes possible to perform focusing control such that a given range including regions forward and rearward of the ideal detection position P of each rice grain is illuminated based on the curvature defining the surface shape of each of the reflectors. Differently from the illuminating device using the conventional reflector defined by the concave curved mirror surface as shown in the first and second embodiments, the illuminating devices according to the sixth to ninth embodiments are free from the problem that a detection image becomes dark or a shadow appears in the image, even if the position of the object surface of each rice grain somewhat deviates. This makes it possible to produce a sorting machine free from deterioration in detection accuracy.

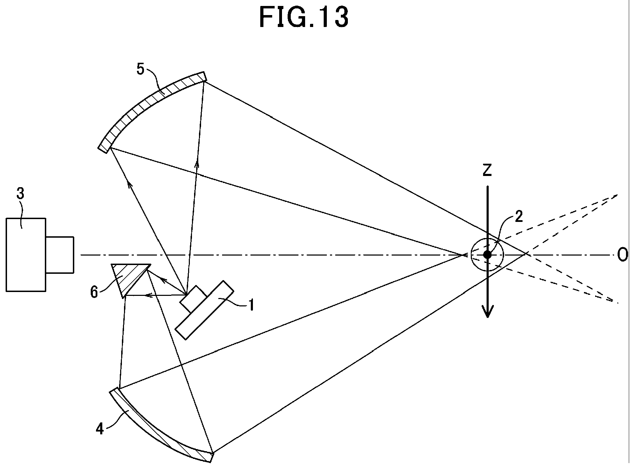

Needless to say, as long as light is focused, e.g., on the rear side of the rice grain as shown in FIG. 13 even in the case where a reflector defined by the concave curved surface described in the embodiments other than the sixth to ninth embodiments is used, it can be expected to illuminate the object surface of the rice grain with light.

On the other hand, the reflectors in the sixth to ninth embodiments provide an excellent effect of being able to control the distribution of luminous energy with respect to each incident angle of a light ray traveling toward a to-be-detected object.

Thus, the use of the reflector defined by the concave curved surface as shown in the sixth to ninth embodiments provides a feature of being able to perform illumination covering the entire object surface of a to-be-detected object, while adequately controlling the distribution of luminous energy even when the to-be-detected object does not fall down along an assumed ideal trajectory. This leads to a situation where a defective part on a to-be-detected object such as a rice grain is clearly recognized as a defective part in a detection image without being hidden by a shadow, thereby providing an effect of being able to significantly improve a recognition rate of defective objects.

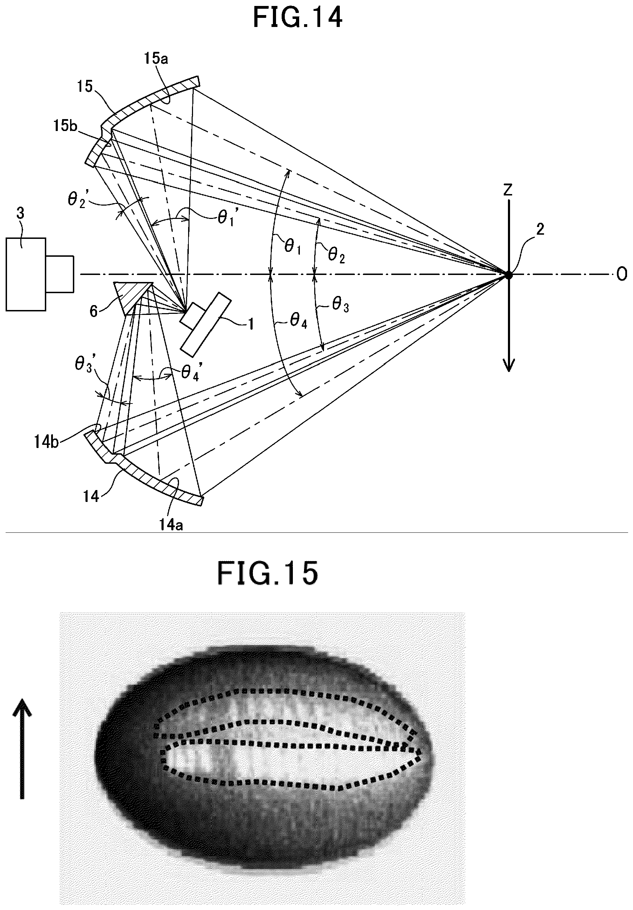

Further, an advantage of composing the reflector of the first concave curved surface portion and the second concave curved surface portion includes a capability of controlling luminous energy values from the concave curved surface portions to become different from each other. In FIG. 14, a surface area ratio between the first concave curved surface portion 14a or 15a and the second concave curved surface portion 14b or 15b illustrated in FIG. 9 is changed for the purpose of explaining the luminous energy control.

As shown in FIG. 14, light rays emitted from the illumination light source and reflected by each of the first concave curved surface portions 14a, 15a farther away from the optical axis O illuminate the rice grain 2 as a to-be-detected object, at a relatively large incident angle (θ₁, θ₄). Further, light rays emitted from the illumination light source and reflected by each of the second concave curved surface portions 14b, 15b closer to the optical axis O illuminate the rice grain 2 at a relatively small incident angle (θ₂, θ₃). Differently from an object such as a rice grain, particularly in a light-impermeable to-be-detected object, it has been found from an experimental result obtained by the present applicant that, if source light is radiated at a relatively small incident angle, a large shadow appears in the vicinity of the contour of the to-be-detected object in a detection image.

Therefore, light rays emitted from the illumination light source are reflected by each of the first concave curved surface portions 14a, 15a farther away from the optical axis O at a larger radiation angle (θ₁', θ₄'). This makes it possible to reduce the shadow which would otherwise appear in the vicinity of the contour of the to-be-detected object. A significant effect is exhibited by using such a reflector in a particular situation where a detection image having a shadow in the vicinity (particularly, upper and lower areas) of the contour of a to-be-detected object is acquired.

FIGS. 9 to 12 show an example in which each of the first reflector 14, 24 or 34 and the second reflector 15, 25 or 35 is configured to spread the focusing position such that an extended region including a to-be-detected object becomes an illumination range. However, the present invention is not intended to exclude a configuration in which this technical idea is applied to only one of the first and second reflectors. Further, in the sixth embodiment, the reflector is defined by the two concave curved surface portions (the first concave curved surface portion and the second concave curved surface portion). However, the present invention is not limited thereto. That is, the reflector may have a larger number of concave curved surface portions. For example, the entire concave curved surface may be composed of three concave curved surface portions.

Further, in a to-be-detected object in which a shadow does not largely appear in upper and lower areas of the contour thereof, the angles θ₁, θ₂, θ₃, θ₄, θ₁', θ₂', θ₃', θ₄' may be set such that luminous energy reflected by each of the first concave curved surface portions 14a, 15a becomes equal to or less than energy reflected by each of the second concave curved surface portions 14b, 15b, and the concave curved surface may be formed such that each of the surface portions has a surface area corresponding thereto.

### < TENTH EMBODIMENT >

Next, an embodiment will be described in which a plurality of lines are added to at least one of the concave curved surfaces of the first reflector and the second reflector.

When a to-be-detected object whose surface has a curvature and is lustrous or glossy is imaged by a camera (e.g., line sensor camera) to obtain a detection image, a light-emitting face of an illumination light source may be undesirably reflected on the detection image (see the area surrounded by the dotted line in FIG. 15). For example, in a case where the illumination light source is constructed by using a plurality of LED chips as light emitters, the array of light emitters are identified on the surface of the to-be-detected object in a recognizable manner, and light indicative of the undesirable reflection is superimposed on illumination light. This is because, if light from the illumination light source is reflected by the first and second reflectors, and then specularly reflected in a specific location of the to-be-detected object whose surface has a curvature, the reflected light is received by the camera. This influence is serious particularly in a to-be-detected object having a glossy surface. In order to enhance detection accuracy, it is necessary to avoid a situation where the LED chips are come out (i.e., identified) on the surface of the to-be-detected object.

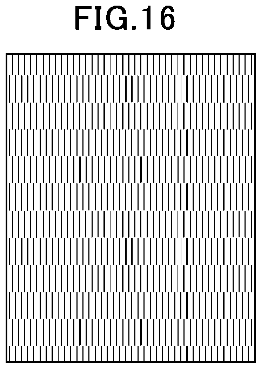

For this purpose, in the illuminating device according to the tenth embodiment, a concave curved mirror for each of the first reflector and the second reflector is formed by concavely curvedly bending an aluminum rolled material such that lines on the surface thereof extending in a rolling direction become longitudinal lines. Alternatively, the concave curved mirror is formed by adding a large number of lines like those obtained through hairline processing, to an aluminum plate having a mirror surface, in a longitudinal direction (see FIG. 16), and then concavely curvedly bending the aluminum plate. Alternatively, in a case where each of the first reflector and the second reflector is produced through resin molding, the concave curved mirror is formed by fabricating longitudinal lines on a metal mold to allow a reflection surface of the concave curved mirror to be formed with longitudinal grooves, and then vapor-depositing aluminum on the surface of the metal mold having the longitudinal lines.

The presence of the longitudinal lines in the concave curved surface of each of the first reflector and the second reflector produces an effect of allowing light emitted from the illumination light source and then reflected by each of the concave curved mirrors to be laterally scattered. This makes it possible to laterally disperse the refraction of the light-emitting face of the LED chips on the surface of the to-be-detected object.

Alternatively, dimples (depressions) having an arbitrarily-set aspect ratio may be formed instead of lines as mentioned above. This produces an effect of allowing light emitted from the illumination light source and then reflected by each of the concave curved mirrors to be scattered not only in the lateral direction but also in the longitudinal direction. This makes it possible to spread the refraction of the light-emitting face of the LED chips on the surface of the to-be-detected object, laterally and longitudinally. In this case, luminous energy directed toward the to-be-detected object is significantly reduced. However, in a situation where there is sufficient luminous energy from LEDs, the effect as means to avoid the undesirable reflection indicative of the LED chips presence is significantly large.

In any of the illuminating devices according to the above embodiments, any light emitter may be used as the illumination light source. For example, when using an LED chip, in a conventional illuminating device, a large number of LED chips are used, and a large number of corresponding driving circuits are used to detect LED burn-out by means of lighting. Thus, it is necessary to spend a lot of cost for the LED chips and the LED driving circuits. The LED chips can be turn on by using a parallel circuit to reduce the number of the LED driving circuits, thereby achieving a reduction in cost. Hoverer, in this case, there is a problem that the LED burn-out cannot be detected.

On the other hand, in the present invention, one illumination light source can be sharedly used for upper and lower illuminations, so that the number of not only the illumination light sources but also the LED chips can be simply reduced to one-half, and the number of the LED driving circuits can also be reduced. Therefore, it becomes possible to realize detection of the LED burn-out while facilitating a reduction in cost.

Further, even though one illumination light source is sharedly used for upper and lower illuminations, luminous energy of illumination light onto a to-be-detected object is two times or more that of a conventional illuminating device using no reflector. Thus, a part of the illumination light source composed of white LEDs can be replaced by an IR LED. In this case, light having a plurality of wavelengths can be simultaneously emitted by one illuminating device. In either case, luminous energy equal to or greater than that of the conventional illuminating device can be obtained using a less number of chips. Thus, a cost reduction effect when using an IR LED more costly than a white LED is significantly large.

### (INDUSTRIAL APPLICABILITY)

Although the above embodiments have been described based on an example in which the present invention is applied to a sorting machine subject to rice grains, the present invention is not necessarily limited thereto. For example, as a subject of sorting by a sorting machine using the present invention, it is possible to use: grains other than rice grains, such as soybeans; granular solids such as coffee beans or seeds; green tea leafs; tablets; and resin pellets containing color paint, which are obtained by processing a synthetic resin such as a bumper of an automobile into a pellet shape in a recycle process.

Further, in a case where the to-be-detected objects are, e.g., sheet-shaped or film-shaped objects stacked on a conveyer, instead of the granular objects such as rice grains, it is possible to observe the sheet-shaped objects or the like from a vertical direction by a camera, and, when a foreign matter is detected in one of the sheet-shaped objects, drive conveying path changing means on the conveyer to convey only the sheet-shaped object containing the foreign matter to a storage casing for irregular objects.

### LIST OF REFERENCE SIGNS

1: illumination light source
2, 2', 2": rice grain
3, 3a, 3b: camera
4, 4': first reflector
5, 5': second reflector
6, 6': refractive mirror
6a, 6a': first reflection surface
6b, 6b': second reflection surface
7: first reflector
7a, 7b, 7c: reflection surface portion
8: first reflector
8a, 8b, 8c: reflection surface portion
9a, 9b: background
14, 24, 34: first reflector
15, 25, 35: second reflector
14a, 15a: first concave curved surface portion
14b, 15b: second concave curved surface portion
Z: falling trajectory of to-be-detected object
O: optical axis
P: ideal detection position
P': detection position deviating on side of front camera 3a
P": detection position deviating on side of rear camera 3b

## Claims

1. An illuminating device used for a sorting machine or an inspecting machine for distinguishing, based on an image of a to-be-detected object detected by an optical detection unit, whether the to-be-detected object is non-defective or defective, comprising:
a light source unit having a light source configured to emit illumination light;
and
a plurality of reflectors configured to reflect light rays from the light source unit toward the to-be-detected object.

2. The illuminating device as recited in claim 1, wherein the plurality of reflectors include a first reflector and a second reflector which are arranged across an optical axis of the optical detection unit, wherein at least one of the first reflector and the second reflector is a concave curved mirror.

3. The illuminating device as recited in claim 2, wherein light rays from the same light source unit are reflected by the first reflector and the second reflector, such that the light rays take respective optical paths toward the to-be-detected object to illuminate the to-be-detected object from thereabove and therebelow.

4. The illuminating device as recited in claim 2 or 3, wherein the light source unit is disposed in a spatial region including the optical axis between the first reflector and the second reflector.

5. The illuminating device as recited in claim 4, wherein a refractive mirror is disposed on optical paths of at least a part of the light rays emitted from the light source unit, and wherein the refractive mirror has a first reflection surface configured to bend and direct the optical paths of at least the part of the light rays toward the first reflector, wherein at least a part of light rays which do not reach the refractive mirror, among the light rays emitted from the light source unit, are incident on the second reflector.

6. The illuminating device as recited in claim 4, wherein a refractive mirror is disposed on optical paths of the light rays emitted from the light source unit, and wherein the refractive mirror has a first reflection surface, and a second reflection surface for changing optical paths to directions different from those in the first reflection surface, wherein
the first reflection surface is configured to bend optical paths of a part of the light rays emitted from the light source unit to reflect the part of the light rays toward the first reflector, and
the second reflection surface is configured to reflect, toward the second reflector, at least a part of light rays which do not reach the first reflection surface, among the light rays from the light source unit.

7. The illuminating device as recited in any one of claims 2 to 6, wherein the concave curved mirror has a first concave curved surface portion defined by a first curvature, and a second concave curved surface portion defined by a second curvature different from the first curvature, wherein the concave curved mirror has a concave shape in which an entirety of a concave surface thereof is composed of the first concave curved surface portion and the second concave curved surface portion.

8. The illuminating device as recited in any one of claims 2 to 6, wherein the concave curved mirror is a concave free-curved mirror whose local curvature radius continuously changes depending on position and orientation on a concave curved surface thereof.

9. The illuminating device as recited in any one of claims 2 to 6, wherein the concave curved mirror is a concave curved mirror whose concave curved surface is approximated by a planar surface.

10. The illuminating device as recited in any one of claims 2 to 9, wherein at least one of the first reflector and the second reflector has a surface shape processed such that a plurality of grooves overlap each other along a longitudinal direction of a concave curved surface thereof.

11. An illuminating device used for a sorting machine or an inspecting machine for distinguishing, based on an image of a to-be-detected object detected by optical detection unit, whether the to-be-detected object is non-defective or defective, comprising:
a light source unit having a light source configured to emit illumination light;
and
a plurality of reflectors configured to reflect light rays from the light source unit toward the to-be-detected object, wherein the plurality of reflectors include a first reflector and a second reflector which are arranged across an optical axis of the optical detection unit, wherein each of the first reflector and the second reflector is a planar mirror.

12. The illuminating device as recited in claim 11, which further comprises a refractive mirror disposed on optical paths of at least a part of the light rays emitted from the light source unit, wherein the refractive mirror has a first concave curved reflection surface and a second concave curved reflection surface, wherein: the first concave curved reflection surface is configured to bend the optical paths of at least the part of the light rays emitted from the light source unit to reflect at least the part of the light rays toward the first reflector; and the second concave curved reflection surface is configured to reflect, toward the second reflector, at least a part of light rays which do not reach the first concave curved reflection surface, among the light rays from the light source unit, whereby the light rays reflected by the first reflector and the second reflector take respective optical paths toward the to-be-detected object to illuminate the to-be-detected object from thereabove and therebelow.

13. The illuminating device as recited in any one of claims 5 to 12, wherein the first reflector and the refractive mirror is formed as a single-structured mirror body.
